# EUROPEAN PATENT APPLICATION

(11) **EP 4 324 820 A1**
(43) Date of publication of application: **21.02.2024**
(21) Application number: 22920069.6
(22) Date of filing: 27.12.2022
(51) Int. Cl.: C07C 209/28, C07C 211/08

(54) **METHOD FOR PREPARING TERTIARY AMINE BY USING SECONDARY AMINE AS RAW MATERIAL**

(30) Priority: 13.01.2022 CN 202210034548
(71) Applicant: Zhejiang Xinhua Chemical Co., Ltd., Zhejiang 311600 (CN); Zhejiang University, Hangzhou, Zhejiang 310058 (CN)
(72) Inventor: HONG, Xin, Zhejiang 311600 (CN); WANG, Yong, Hangzhou, Zhejiang 310027 (CN); YING, Sibin, Zhejiang 311600 (CN); LIU, Zhen, Zhejiang 311600 (CN); LIU, Chengwei, Zhejiang 311600 (CN); HONG, Xu, Zhejiang 311600 (CN); CHEN, Jieya, Zhejiang 311600 (CN); XU, Ke, Zhejiang 311600 (CN)
(74) Representative: Cabinet Bleger-Rhein-Poupon
(86) International application number: PCT/CN2022/142367
(87) International publication number: WO 2023/134439

(57) **Abstract**

Disclosed in the present invention is a method for preparing a tertiary amine by using a secondary amine as a raw material. The method comprises a step of reacting a secondary amine that serves as a raw material with an acid to generate a proton-type ionic liquid, and a step of reacting the proton-type ionic liquid with an aldehyde under the action of a reducing agent to generate a tertiary amine. The secondary amine is an aliphatic secondary amine and/or an aromatic secondary amine and/or a cyclic secondary amine; the acid is selected from one of or a combination of several of an organic carboxylic acid and an inorganic acid; the aldehyde is a monoaldehyde, a dialdehyde or an aldehyde polymer; and the reducing agent is selected from one of or a combination of several of formic acid, sodium formate, oxalic acid, sodium oxalate and triphenylsilane. The proton-type ionic liquid in the present invention can play the role of autocatalyzing the reaction, such that the high yield and high purity of the tertiary amine product are ensured, moreover, the reaction conditions are mild, high pressure and a hydrogen reduction atmosphere are not required, and a safe and green reducing agent can be used.

## Description

### Tchnical Field of the Invention

The present disclosure relates to a method of preparing a tertiary amine with a secondary amine as raw material.

### Background of the Invention

Tertiary amines are widely used in industrial production and life, and can be used as fuel additives, pesticides, pharmaceuticals, epoxy resin hardeners, intermediates of polyurethane catalysts, raw materials for the preparation of quaternary ammonium salts/ bases, plasticizers, dyes, desulfurizers, etc..

It is known that the traditional synthesis of tertiary amine products often uses a high-pressure fixed bed reactor, and uses heterogeneous noble metal catalysts such as Pd and Pt supported on the support, and the reactions also need to be carried out under hydrogen conditions. For example, CN101460445A discloses a method for preparing diisopropylethylamine by amination of diisopropylamine and acetaldehyde using a suspended catalyst Pd/C as catalyst, the reaction requires high pressure and hydrogen conditions, and the noble metal catalyst is expensive.

It is also known that secondary amines and aldehydes with more than two carbon atoms can be used to react to generate tertiary amines in the presence of reducing agents under atmospheric pressure without using metal catalysts and hydrogen. For example, published Chinese patent CN101360726A discloses a method for preparing a tertiary amine by dropping a secondary amine into a mixed solution of an aldehyde and an acid, and this method is characterized by mixing the aldehyde and formic acid first, heating to reflux temperature, and then adding the secondary amine for reaction. This method requires the dropping sequence of raw materials, and when the aldehyde or acid is dropped into the other two raw materials, the reaction yield is significantly reduced. After the reaction, a large amount of alkali (such as NaOH aqueous solution) needs to be added to the reaction system to neutralize the reaction system, causing the reaction produce a large amount of wastewater containing organic substances.

It is also known that published Chinese patent CN102875385A discloses a method of using paraldehyde as raw material, hydrolyzing with an acid catalyst, and then reacting with diisopropylamine and a reducing agent metal hydride such as sodium borohydride to generate a tertiary amine. This patent does not disclose the purity of the tertiary amine product, meanwhile, the reducing agent used, such as sodium borohydride, is highly toxic, easy to explode, sensitive to water, and not conducive to the operation of the reaction.

### Summary of the Invention

The technical problem to be solved by the present disclosure is to overcome the defects and deficiencies of the prior art, and provides an improved method for preparing a tertiary amine by the reaction of a protic ionic liquid and an aldehyde, which is a homogeneous autocatalytic reaction, does not require high-pressure hydrogen and metal catalyst conditions, can use safe and green reductants, and has safer addition process of reaction materials.

To solve the above technical problems, a technical solution employed by the present disclosure is as follows:
A method for preparing a tertiary amine with a secondary amine as raw material, which comprises the step of reacting a secondary amine as raw material with an acid to generate a protic ionic liquid, and the step of reacting the protic ionic liquid with an aldehyde under the action of a reducing agent to generate a tertiary amine.

Further, the secondary amine has a carbon number below 30, preferable below 25, and further preferably below 20; and/or, the secondary amine is an aliphatic secondary amine and/or an aromatic secondary amine; and/or, the acid is selected from the group consisting of organic carboxylic acids, inorganic acids, and combinations thereof; and/or, the aldehyde is a monoaldehyde, a dialdehyde, or an aldehyde polymer.

Further, the acid is selected from the group consisting of C₁ - C₃ organic carboxylic acids, hydrochloric acid, sulfuric acid, phosphoric acid, nitric acid, and combinations thereof; preferably, the acid is selected from the group consisting of formic acid, acetic acid, propionic acid, oxalic acid, malonic acid, carbonic acid, hydrochloric acid, sulfuric acid, phosphoric acid, nitric acid, and combinations thereof.

In some implementations of the present disclosure, the reducing agent is selected from the group consisting of formic acid, sodium formate, oxalic acid, sodium oxalate, triphenyl silane, and combinations thereof; preferably, the reducing agent is selected from the group consisting of formic acid, sodium formate, oxalic acid, and sodium oxalate.

The inventors found that secondary amines and acids can react at room temperature and atmospheric pressure to generate protic ionic liquids, with safe and controllable operation, where the molar ratio of secondary amines and acids is close to 1, and the consumption of acids is small. By reacting the protic ionic liquids with aldehydes under the action of reducing agents to generate tertiary amines, it can be achieved that the reaction conditions for reacting secondary amines and aldehydes to generate tertiary amines are mild, no high-pressure or hydrogen reduction atmosphere is required during the reaction process, and the reaction yield is high, the purity of the tertiary amines obtained is high, and safe and green reducing agents are used in the reaction system. The protic ionic liquid generated in the reaction system can play a catalytic role in the reaction, and the reaction system of the present dislcosure is an autocatalytic reaction system, and there is no need to add metal catalyst.

In some implementations of the present disclosure, the molar ratio of the secondary amine as raw material to the acid is 0.2 - 1.5, preferably 0.3 - 1.4, especially preferably 0.4 - 1.2.

In some implementations of the present disclosure, the molar ratio of the aldehyde to the protic ionic liquid is 0.2 - 5.0, preferably 0.3 - 3.0, especially preferably 0.4 - 2.0.

In some implementations of the present disclosure, the molar ratio of the reducing agent to the protic ionic liquid is 1.0 - 5.0, preferably 1.0 - 2.0.

In some implementations of the present disclosure, the step of reacting a secondary amine as raw material with an acid to generate a protic ionic liquid is carried out at room temperature and atmospheric pressure.

In some implementations of the present disclosure, the step of reacting the protic ionic liquid with an aldehyde under the action of a reducing agent to generate a tertiary amine is carried out at a pressure of 0 - 5.0 MPa, preferably 0 - 3.0 MPa, and particularly preferably 0 - 2.0 MPa.

In some implementations of the present disclosure, the step of reacting the protic ionic liquid with an aldehyde under the action of a reducing agent to generate a tertiary amine is carried out at a temperature of 60 - 200°C, preferably 120 - 160°C.

In some implementations of the present disclosure, the reaction time of the protic ionic liquid and the aldehyde under the action of a reducing agent is 5 - 300 min, preferably 30 - 120 min.

In some implementations of the present disclosure, the method comprises the following steps:
1) dropwise adding the acid to the secondary amine or the secondary amine to the acid to generate the protic ionic liquid;
2) adding the protic ionic liquid and the reducing agent to a reactor, and adding the aldehyde to the reactor;
3) introducing nitrogen to the reaction system to a pressure of 0 - 5.0 MPa, heating to 60 - 200°C, and reacting for 5 - 300 min;
4) after the reaction is completed, purifying the reaction system to give the tertiary amine.

In some implementations of the present disclosure, in step 1), the acid is added dropwise to an aqueous solution of the secondary amine to generate the protic ionic liquid.

In some implementations of the present disclosure, in step 1), the acid is added dropwise to the secondary amine in the form of an aqueous solution of the acid.

In some implementations of the present disclosure, in step 2), the aldehyde is added at once, intermittently, or continuously into the reactor.

In some implementations of the present disclosure, in step 2), the reducing agent is added at once, intermittently, or continuously into the reactor. In some implementations of the present disclosure, in step 4), the purification can be carried out through atmospheric distillation or reduced pressure distillation according to the boiling point of the product.

In some implementations of the present disclosure, the reactor employs a stirring vessel, an autoclave, a tubular reactor, a micro reactor, or a packed bubble tower, preferably an autoclave.

Further, the structural formula of the secondary amine is 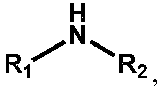 where R₁ and R₂ are independently selected from the group consisting of C₁ - C₈ alkyl, C₄ - C₈ cycloalkyl, aryl, and C₇ - C₉ alkaryl, or R₁, R₂ and NH together form a C₃ - C₇ ring; and/or the aldehyde is selected from the group consisting of monoaldehydes with a structural formula 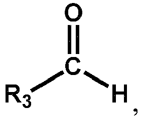 polymers of monoaldehydes with a structural formula 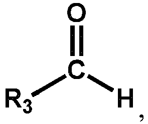 and dialdehydes with a structural formula 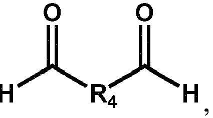 where R₃ is selected from the group consisting of H, C₁ - C₆ alkyl, and hydroxy substituted C₁ - C₆ alkyl, and R₄ is a single bond or C₁ - C₄ alkylidene.

The structural formula of the protic ionic liquid is 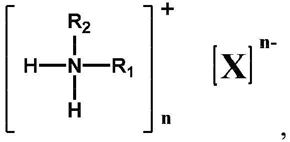 where R₁ and R₂ are independently selected from the group consisting of C₁ - C₈ alkyl, C₄ - C₈ cycloalkyl, aryl, and C₇ - C₉ alkaryl, or R₁, R₂ and N together form a C₃ - C₇ ring, n is 1, 2 or 3, and [X]ⁿ⁻ is selected from the group consisting of C₁-C₃ organic carboxyl, CO₃² , HCO₃⁻, Cl, NO₃⁻, SO₄²⁻, PO₄³⁻, HPO₄²⁻ and H₂PO₄⁻.

When the aldehyde is selected from monoaldehydes with a structural formula 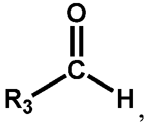 and polymers of monoaldehydes with a structural formula 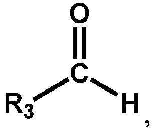 the reaction equation of the protic ionic liquid and the aldehyde is shown below: 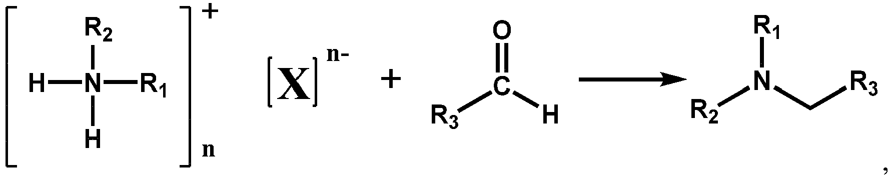 when the aldehyde is selected from dialdehydes with a structural formula 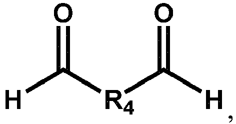 the reaction equation of the protic ionic liquid and the aldehyde is shown below:

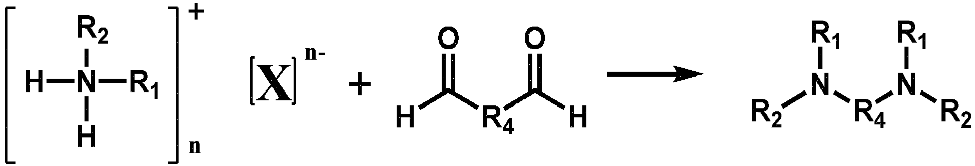

The protic ionic liquid first reacts with the aldehyde to generate a corresponding Schiff base, which is reduced by the reducing agent to give the tertiary amine.

In some implementations of the present disclosure, R₁ and R₂ are independently selected from the group consisting of methyl, ethyl, n-propyl, isopropyl, n-butyl, isobutyl, sec-butyl, tert-butyl, n-pentyl, isopentyl, sec-pentyl, neopentyl, 1,2-dimethylpropyl, n-hexyl, isohexyl, sec-hexyl, n-heptyl, isoheptyl, sec-heptyl, n-octyl, isooctyl, sec-octyl, 2-ethylhexyl, cyclopropyl, cyclobutyl, cyclopentyl, cyclohexyl, cycloheptyl, cyclooctyl, phenyl, 2-naphthyl, 2-methyl phenyl, 3-methyl phenyl, 4-methyl phenyl, 2,4-dimethyl phenyl, 2,5-dimethyl phenyl, 2,6-dimethyl phenyl, 3,4-dimethyl phenyl, 3,5-dimethyl phenyl, 2,3,4-trimethyl phenyl, 2,3,5-trimethyl phenyl, 2,3,6-trimethyl phenyl, 2,4,6-trimethyl phenyl, 2-ethyl phenyl, 3-ethyl phenyl, 4-ethyl phenyl, 2-n-propyl phenyl, 3-n-propyl phenyl, and 4-n-propyl phenyl.

In some other implementations of the present disclosure, R₁, R₂ and NH together form azetidine, pyrrole, piperidine, hexamethyleneimine, morpholine, piperazine or N-methylpiperazine.

In some other implementations of the present disclosure, R₃ is selected from the group consisting of H, methyl, ethyl, n-propyl, isopropyl, n-butyl, isobutyl, n-pentyl, isopentyl, n-hexyl, isohexyl, hydroxymethyl, hydroxyethyl, and hydroxypropyl.

In some implementations of the present disclosure, R₄ is selected from the group consisting of a single bond, methylene, ethylidene, propylidene, and butylidene.

In some implementations of the present disclosure, the aldehyde is selected from the group consisting of acetaldehyde, paraldehyde, metaldehyde, propionaldehyde, glycolaldehyde, glyoxal, malondialdehyde, succinaldehyde, glutaraldehyde and adipaldehyde.

In some implementations of the present disclosure, the secondary amine is selected from the group consisting of dimethylamine, diisopropylamine, piperidine, morpholine and N-methylpiperazine; the aldehyde is selected from the group consisting of acetaldehyde, paraldehyde, metaldehyde, propionaldehyde, glycolaldehyde, and succinaldehyde; the tertiary amine is selected from the group consisting of dimethylethylamine, dimethylpropylamine, N,N,N',N'-tetramethylbutanediamine, diisopropylethylamine, diisopropyl aminoethanol, N-ethylpiperidine, N-propylmorpholine, N-propylpiperazine and N, N'-dipropylpiperazine.

In a particularly preferred implementation of the present disclosure, acetaldehyde reacts with dimethylamine to give dimethylethylamine.

In a particularly preferred implementation of the present disclosure, propionaldehyde reacts with dimethylamine to give dimethylpropylamine.

In a particularly preferred implementation of the present disclosure, succinaldehyde reacts with dimethylamine to give N,N,N',N'-tetramethylbutanediamine.

In a particularly preferred implementation of the present disclosure, acetaldehyde reacts with diisopropylamine to give diisopropylethylamine.

In a particularly preferred implementation of the present disclosure, paraldehyde reacts with diisopropylamine to give diisopropylethylamine.

In a particularly preferred implementation of the present disclosure, glycolaldehyde reacts with diisopropylamine to give diisopropyl aminoethanol.

In a particularly preferred implementation of the present disclosure, acetaldehyde reacts with piperidine to give N-ethylpiperidine.

In a particularly preferred implementation of the present disclosure, propionaldehyde reacts with morpholine to give N-propylmorpholine.

In a particularly preferred implementation of the present disclosure, propionaldehyde reacts with piperazine to give N-propylpiperazine.

In a particularly preferred implementation of the present disclosure, propionaldehyde reacts with piperazine to give N, N'-dipropylpiperazine.

In a particularly preferred implementation of the present disclosure, acetaldehyde reacts with dicyclohexylamine to give N-ethyldicyclohexylamine.

In a particularly preferred implementation of the present disclosure, acetaldehyde reacts with diphenylamine to give N-ethyldiphenylamine.

The present disclosure has the following advantages over the prior art:
The present disclosure creatively introduces a system in which secondary amines are used as raw materials to generate tertiary amines, and in which, firstly, acids are added to react with secondary amines to generate protic ionic liquids, and then the protic ionic liquids react with aldehydes to generate tertiary amines, the protic ionic liquids can play a self-catalytic role, ensuring high purity and yield of tertiary amine products while achieving mild reaction conditions without the need for high pressure or hydrogen reaction atmosphere, and gentle and safe reducing agents can be used. The purity of the tertiary amine products obtained by the present disclosure reach 99%, and the yield reach 98%. The reaction is a homogeneous autocatalytic reaction, and the production cost is greatly reduced.

### Brief Description of the Drawings

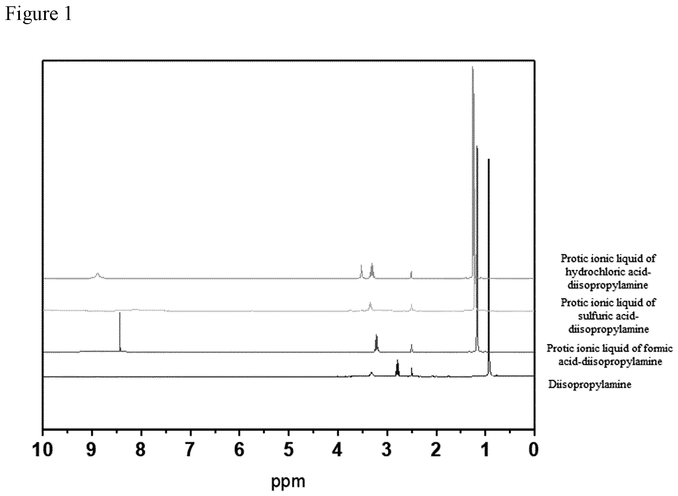

Figure 1 shows the nuclear magnetic spectrums of diisopropylamine and corresponding protic ionic liquids formed from diisopropylamine with formic acid, hydrochloric acid, and sulfuric acid respectively;
wherein, diisopropylamine: δ_{H}(DMSO) 0.93(d, 12H), 2.80(m, 2H), 3.32(s, 2H).

The protic ionic liquid of formic acid-diisopropylamine: δ_{H}(DMSO) 1.18(d, 12H), 3.31(m, 2H), 8.80(s, 1H).

The protic ionic liquid of sulfuric acid-diisopropylamine: δ_{H}(DMSO) 1.22(d, 12H), 3.34(m, 2H), 8.13(s, 2H).

The protic ionic liquid of hydrochloric acid-diisopropylamine: δ_{H}(DMSO) 1.25(d, 12H), 3.31(m, 2H), 8.89(s, 2H).

### Detailed Description of Exemplary Embodiments

The present disclosure is further described below combining with embodiments. But the present disclosure is not limited to the embodiments below. The implementation conditions used in the embodiments may be further adjusted according to different requirements of specific use, and undefined implementation conditions usually are conventional conditions in the industry. The technical features involved in the various embodiments of the present disclosure may be combined with each other if they do not conflict with each other.

Various raw materials used in the following embodiments were purchased from Aladdin Reagents company, wherein dimethylamine was an aqueous solution with a purity of 40%, diisopropylamine, piperidine, morpholine, ethylamine, dicyclohexylamine, paraldehyde, glycolaldehyde, succinaldehyde, acetic acid, propionic acid and malonic acid were aqueous solutions with a purity of ≥ 99%, piperazine, diphenylamine, sodium formate, oxalic acid and sodium oxalate were solids with a purity of ≥ 99%, formic acid was a solid with a purity of ≥ 98%, hydrochloric acid was an aqueous solution with a purity of ≥ 36.5%, phosphoric acid was an aqueous solution with a purity of ≥ 85%, acetaldehyde, propionaldehyde, and concentrated sulfuric acid were aqueous solutions with a purity of ≥ 98%, and concentrated nitric acid was an aqueous solution with a purity of ≥ 65%.

### Embodiment 1

### Synthesis of dimethylethylamine by reacting dimethylamine and acetaldehyde:

In a 100 mL high-pressure reactor, 15 g of 40% dimethylamine aqueous solution was added, 7.5 g of formic acid solution was added dropwise to the reactor, and stirred to form a uniform solution, to prepare an aqueous solution of the protic ionic liquid of formic acid-dimethylamine, then 10 g of sodium formate solid and 6.5 g of acetaldehyde solution were added to the aqueous solution of the protic ionic liquid, the reactor was sealed, nitrogen was introduced to increase the pressure to 1 MPa, and the temperature was raised to 140°C to react for 120 min.

The reaction product mixture was purified by atmospheric distillation, fractions at 34 to 36°C were collected, the fractions were analyzed for purity using gas chromatography, and the measurement program used in gas chromatography was: separation column HP-1, length: 60 m, inner diameter: 0.25 mm, carrier: nitrogen, temperature program: 55°C, then increase 10°C every minute to 220°C, and finally maintain a constant temperature at 220°C for 10 min to give dimethylethylamine with a purity of 99%, and a product yield of 95%.

### Embodiment 2

### Synthesis of dimethylpropylamine by reacting dimethylamine and propionaldehyde:

In a 100 mL high-pressure reactor, 15 g of 40% dimethylamine aqueous solution was added, 8 g of acetic acid solution was added dropwise to the reactor, and stirred to form a uniform solution, to prepare an aqueous solution of the protic ionic liquid of acetic acid-dimethylamine, then 10 g of sodium formate solution and 8 g of propionaldehyde solution were added to the aqueous solution of the protic ionic liquid, the reactor was sealed, nitrogen was introduced to increase the pressure to 2 MPa, and the temperature was raised to 160°C to react for 120 min.

The reaction product mixture was purified by atmospheric distillation, fractions at 70 - 71°C were collected, and the fractions were analyzed for purity using gas chromatography using the same method as Embodiment 1, to give dimethylpropylamine with a purity of 99%, and a product yield of 96%.

### Embodiment 3

### Synthesis of N, N, N', N'-tetramethylbutanediamine by reacting dimethylamine and succinaldehyde:

In a 100 mL high-pressure reactor, 15 g of 40% dimethylamine aqueous solution was added, 11 g of propionic acid solution was added dropwise to the reactor, and stirred to form a uniform solution, to prepare an aqueous solution of the protic ionic liquid of propionic acid-dimethylamine, then 13 g of oxalic acid and 5 g of succinaldehyde were added to the aqueous solution of the protic ionic liquid, the reactor was sealed, nitrogen was introduced to increase the pressure to 2 MPa, and the temperature was raised to 160°C to react for 120 min.

The reaction product mixture was purified by atmospheric distillation, fractions at 166 - 167°C were collected, and the fractions were analyzed for purity using gas chromatography using the same method as Embodiment 1, to give N, N, N', N'-tetramethylbutanediamine with a purity of 99%, and a product yield of 95%.

### Embodiment 4

### Synthesis of diisopropylethylamine by reacting diisopropylamine and acetaldehyde:

In a 100 mL high-pressure reactor, 15 g of diisopropylamine was added, 15 g of concentrated hydrochloric acid was added dropwise to the reactor, and stirred to form a uniform solution, to prepare an aqueous solution of the protic ionic liquid of hydrochloric acid-diisopropylamine (its nuclear magnetic spectrum in DMSO is shown in Figure 1), then 10 g of sodium formate and 7 g of acetaldehyde were added to the aqueous solution of the protic ionic liquid, the reactor was sealed, nitrogen was introduced to increase the pressure to 2 MPa, and the temperature was raised to 120°C to react for 30 min.

The reaction product mixture was purified by atmospheric distillation, fractions at 127 - 128°C were collected, and the fractions were analyzed for purity using gas chromatography using the same method as Embodiment 1, to give diisopropylethylamine with a purity of 99%, and a product yield of 97%.

### Embodiment 5

### Synthesis of diisopropylethylamine by reacting diisopropylamine and paraldehyde:

In a 100 mL high-pressure reactor, 15 g of diisopropylamine was added, an aqueous solution containing 7 g of formic acid was added to the reactor, and stirred to form a uniform solution, to prepare an aqueous solution of the protic ionic liquid of formic acid-diisopropylamine (its nuclear magnetic spectrum in DMSO is shown in Figure 1), then 7 g of formic acid and 7 g of paraldehyde were added to the aqueous solution of the protic ionic liquid, the reactor was sealed, nitrogen was introduced to maintain atomospheric pressure (namely the reading of the pressure gauge is 0 MPa), and the temperature was raised to 160°C to react for 120 min.

After the reaction was completed, the oil and water were stratified, the water layer can be directly recycled and reused, the oil was subjected to atmospheric distillation, fractions at 127 - 128°C were collected, and the fractions were analyzed for purity using gas chromatography using the same method as Embodiment 1, to give diisopropylethylamine with a purity of 99%, and a product yield of 98%.

### Embodiment 6

### Synthesis of diisopropyl aminoethanol by reacting diisopropylamine and glycolaldehyde:

In a 100 mL high-pressure reactor, 15 g of diisopropylamine was added, an aqueous solution containing 12 g of concentrated sulfuric acid (10% content) was added dropwise to the reactor, and stirred to form a uniform solution, to prepare an aqueous solution of the protic ionic liquid of sulfuric acid-diisopropylamine (its nuclear magnetic spectrum in DMSO is shown in Figure 1), then 10 g of sodium formate and 9 g of glycolaldehyde were added to the aqueous solution of the protic ionic liquid, the reactor was sealed, nitrogen was introduced to increase the pressure to 2 MPa, and the temperature was raised to 120°C to react for 30 min.

The reaction product mixture was purified by atmospheric distillation, fractions at 187 - 192°C were collected, and the fractions were analyzed for purity using gas chromatography using the same method as Embodiment 1, to give diisopropyl aminoethanol with a purity of 99%, and a product yield of 94%.

### Embodiment 7

### Synthesis of N-ethylpiperidine by reacting piperidine and acetaldehyde:

In a 100 mL high-pressure reactor, 8.5 g of piperidine was added, an aqueous solution containing 10 g of phosphoric acid (10% content) was added dropwise to the reactor, and stirred to form a uniform solution, to prepare an aqueous solution of the protic ionic liquid of phosphoric acid-piperidine, then 5.0 g of formic acid and 4.6 g of acetaldehyde were added to the aqueous solution of the protic ionic liquid, the reactor was sealed, nitrogen was introduced to increase the pressure to 2 MPa, and the temperature was raised to 160°C to react for 90 min.

The reaction product mixture was purified by atmospheric distillation, fractions at 126 - 129°C were collected, and the fractions were analyzed for purity using gas chromatography using the same method as Embodiment 1, to give N-ethylpiperidine with a purity of 99%, and a product yield of 94%.

### Embodiment 8

### Synthesis of N-propylmorpholine by reacting morpholine and propionaldehyde:

In a 100 mL high-pressure reactor, 15 g of morpholine was added, 17 g of concentrated hydrochloric acid was added dropwise to the reactor, and stirred to form a uniform solution, to prepare an aqueous solution of the protic ionic liquid of hydrochloric acid-morpholine, then 12 g of sodium formate and 11 g of propionaldehyde were added to the aqueous solution of the protic ionic liquid, the reactor was sealed, nitrogen was introduced to increase the pressure to 2 MPa, and the temperature was raised to 160°C to react for 120 min.

The reaction product mixture was purified by reduced pressure distillation, fractions at 67 - 69°C at a pressure of 30 mmHg were collected, and the fractions were analyzed for purity using gas chromatography using the same method as Embodiment 1, to give N-propylmorpholine with a purity of 99%, and a product yield of 90%.

### Embodiment 9

### Synthesis of N-propylpiperazine by reacting piperazine and propionaldehyde:

In a 100 mL high-pressure reactor, 15 g of piperazine was added, an aqueous solution containing 18 g of concentrated nitric acid (10% content) was added dropwise to the reactor, and stirred to form a uniform solution, to prepare an aqueous solution of the protic ionic liquid of nitric acid-piperazine, then 12 g of sodium formate was added to the aqueous solution of the protic ionic liquid, the reactor was sealed, nitrogen was introduced to increase the pressure to 2 MPa, and 10 g of propionaldehyde was added within 1 hour after the temperature was raised to 160°C and reacted for 120 min.

The reaction product mixture was purified by reduced pressure distillation, fractions at 61 - 64°C at a pressure of 15 mmHg were collected, and the fractions were analyzed for purity using gas chromatography using the same method as Embodiment 1, to give N-propylpiperazine with a purity of 99%, and a product yield of 91%.

### Embodiment 10

### Synthesis of N,N'-dipropylpiperazine by reacting piperazine and propionaldehyde:

In a 100 mL high-pressure reactor, 15 g of piperazine was added, an aqueous solution containing 18 g of concentrated nitric acid (10% content) was added dropwise to the reactor, and stirred to form a uniform solution, to prepare an aqueous solution of the protic ionic liquid of nitric acid-piperazine, then 24 g of sodium formate and 20 g of propionaldehyde were added to the aqueous solution of the protic ionic liquid, the reactor was sealed, nitrogen was introduced to increase the pressure to 2 MPa, and the temperature was raised to 160°C to react for 120 min.

The reaction product mixture was purified by reduced pressure distillation, fractions at 84 - 88°C at a pressure of 12 mmHg were collected, and the fractions were analyzed for purity using gas chromatography using the same method as Embodiment 1, to give N, N'-dipropylpiperazine with a purity of 99%, and a product yield of 90%.

### Embodiment 11

### Synthesis of N, N, N', N'-tetramethylbutanediamine by reacting dimethylamine and succinaldehyde:

In a 100 mL high-pressure reactor, 15 g of 40% dimethylamine aqueous solution was added, 14 g of malonic acid aqueous solution was added dropwise to the reactor, and stirred to form a uniform solution, to prepare an aqueous solution of the protic ionic liquid of malonic acid-dimethylamine, then 13 g of oxalic acid was added to the aqueous solution of the protic ionic liquid, the reactor was sealed, nitrogen was introduced to increase the pressure to 2 MPa, and the temperature was raised to 160°C to react for 120 min, wherein 5 g of succinaldehyde was introduced into the reaction system evenly in the first 60 min.

The reaction product mixture was purified by atmospheric distillation, fractions at 166 - 167°C were collected, and the fractions were analyzed for purity using gas chromatography using the same method as Embodiment 1, to give N, N, N', N'-tetramethylbutanediamine with a purity of 99%, and a product yield of 96%.

### Embodiment 12

### Synthesis of N-ethyldicyclohexylamine by reacting acetaldehyde and dicyclohexylamine:

In a 100 mL high-pressure reactor, 24 g of dicyclohexylamine was added, 14 g of concentrated hydrochloric acid was added dropwise to the reactor, and stirred to form a uniform solution, to prepare an aqueous solution of the protic ionic liquid of hydrochloric acid-dicyclohexylamine, then 10 g of sodium formate and 7 g of acetaldehyde were added to the aqueous solution of the protic ionic liquid, the reactor was sealed, nitrogen was introduced to increase the pressure to 2 MPa, and the temperature was raised to 160°C to react for 120 min.

The reaction product mixture was purified by reduced pressure distillation, fractions at 137 - 138°C at a pressure of 14 mmHg were collected, and the fractions were analyzed for purity using gas chromatography using the same method as Embodiment 1, to give N-ethyldicyclohexylamine with a purity of 99%, and a product yield of 94%.

### Embodiment 13

### Synthesis of N-ethyldiphenylamine by reacting acetaldehyde and diphenylamine:

In a 100 mL high-pressure reactor, 23 g of diphenylamine solid was added, an aqueous solution containing 12 g of concentrated sulfuric acid (10% content) was added dropwise to the reactor, and stirred to form a uniform solution, to prepare an aqueous solution of the protic ionic liquid of sulfuric acid-diphenylamine, then 15 g of oxalic acid and 6 g of acetaldehyde were added to the aqueous solution of the protic ionic liquid, the reactor was sealed, nitrogen was introduced to increase the pressure to 2 MPa, and the temperature was raised to 160°C to react for 120 min.

The reaction product mixture was purified by reduced pressure distillation, fractions at 150 - 152°C at a pressure of 14 mmHg were collected, and the fractions were analyzed for purity using gas chromatography using the same method as Embodiment 1, to give N-ethyldiphenylamine with a purity of 99%, and a product yield of 92%.

### Embodiment 14

### Synthesis of triethylamine by reacting diethylamine and acetaldehyde:

In a 100 mL high-pressure reactor, 10 g of diethylamine was added, 4.5 g of formic acid was added dropwise to prepare an aqueous solution of the protic ionic liquid of formic acid-diethylamine, then 19 g of sodium oxalate solid and 6.5 g of acetaldehyde were added to the aqueous solution of the protic ionic liquid, the reactor was sealed, nitrogen was introduced to increase the pressure to 2 MPa, and the temperature was raised to 120°C to react for 120 min.

After the reaction was completed, the residual gas in the reactor was removed and the reaction product mixture was analyzed using gas chromatography. The gas chromatography was the same as in Embodiment 1.

The gas chromatography showed that the reaction system contained diethylamine, that is, ethylamine and acetaldehyde generated diethylamine under the reduction of sodium formate. The reaction product mixture was purified by atmospheric distillation, fractions at 89 - 91°C were collected to give triethylamine with a purity of 99%, and a product yield of 94%.

### Comparative example 1

### Reaction of diisopropylamine and acetaldehyde:

In a 100 mL high-pressure reactor, 15 g of diisopropylamine was added, then 11g of sodium formate and 7 g of acetaldehyde were added, the reactor was sealed, nitrogen was introduced to increase the pressure to 2 MPa, and the temperature was raised to 120°C to react for 120 min.

After the reaction was completed, the residual gas in the reactor was removed and the reaction product mixture was analyzed using gas chromatography. The gas chromatography was the same as in Embodiment 1. Through gas chromatography analysis, only the sample peaks of acetaldehyde and diisopropylamine were found in the reaction product mixture, indicating that diisopropylethylamine was not generated in the reactor. Comparing this comparative example 1 with Embodiment 4, it can be seen that the acid and the secondary amine added in the present disclosure form a protic ionic liquid, and this protic ionic liquid is easy to react with an aldehyde to form a Schiff base, which is then reduced to a tertiary amine through a reducing agent, and when no acid is added, the system does not form a protic ionic liquid, and the secondary amine and the aldehyde cannot react.

### Comparative example 2

### Synthesis of diethylamine by reacting ethylamine and acetaldehyde:

In a 100 mL high-pressure reactor, 7 g of ethylamine was added, then 23 g of sodium formate solid and 14 g of acetaldehyde were added, the reactor was sealed, nitrogen was introduced to increase the pressure to 2 MPa, and the temperature was raised to 120°C to react for 120 min.

After the reaction was completed, the residual gas in the reactor was removed and the reaction product mixture was analyzed using gas chromatography. The gas chromatography was the same as in Embodiment 1.

The gas chromatography showed that the reaction system contained diethylamine, that is, ethylamine and acetaldehyde generated diethylamine under the reduction of sodium formate. The reaction product mixture was purified by atmospheric distillation, fractions at 55 - 56°C were collected to give diethylamine with a purity of 99%, and a product yield of 55%.

Usually, the reaction of primary amines and aldehydes to generate secondary amines is easier to carry out than the reaction of corresponding secondary amines and aldehydes to generate tertiary amines, manifested as a higher yield of secondary amines generated by the reaction of primary amines and aldehydes, compared to the corresponding yield of tertiary amines generated from secondary amines. However, for the reaction of a primary amine diethylamine and acetaldehyde to generate a secondary amine diethylamine in Comparative example 2, the yield is significantly lower than that of the present disclosure, where diethylamine first reacts with formic acid to form a protic ionic liquid of formic acid-diethylamine, which then reacts with acetaldehyde and is reduced to generate a tertiary amine triethylamine, indicating that in the present disclosure, when the protic ionic liquids react with aldehydes and are reduced to generate tertiary amines, the protic ionic liquids not only participate in the reaction itself, but also has a certain catalytic effect on the reaction.

The embodiments described above are only for illustrating the technical concepts and features of the present disclosure, and are intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

The endpoints and any values of the ranges disclosed herein are not limited to the precise ranges or values, and these ranges or values should be understood to include values close to these ranges or values. For ranges of value, between the end values of each range, between the end values of each range and individual point values, and between individual point values can be combined with each other to obtain one or more new ranges of value, and these ranges of value should be considered as specifically disclosed herein.

## Claims

1. A method for preparing a tertiary amine with a secondary amine as raw material, **characterized in that,** the method comprises the step of reacting a secondary amine with an acid to generate a protic ionic liquid, and the step of reacting the protic ionic liquid with an aldehyde under the action of a reducing agent to generate a tertiary amine; the method comprises the following steps:
1) dropwise adding the acid to the secondary amine or the secondary amine to the acid to generate the protic ionic liquid;
2) adding the protic ionic liquid and the reducing agent to a reactor, and adding the aldehyde to the reactor;
3) injecting nitrogen to the reaction system to a pressure of 0 - 5.0 MPa, heating to 60 - 200°C, and reacting for 5-300 min;
4) after the reaction is completed, purifying the reaction system to give the tertiary amine;
the structural formula of the secondary amine is 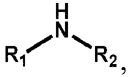 where R₁ and R₂ are independently selected from the group consisting of C₁ - C₈ alkyl, C₄ - C₈ cycloalkyl, aryl, and C₇ - C₉ alkaryl, or R₁, R₂ and NH together form a C₃ - C₇ ring;
the aldehyde is selected from the group consisting of monoaldehydes with a structural formula 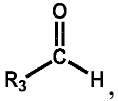 polymers of monoaldehydes with a structural formula 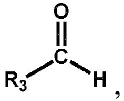 and dialdehydes with a structural formula 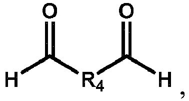 where R₃ is selected from the group consisting of H, C₁ - C₆ alkyl, and hydroxy substituted C₁ - C₆ alkyl, and R₄ is a single bond or C₁ - C₄ alkylidene;
the structural formula of the protic ionic liquid is 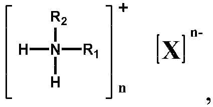 where R₁ and R₂ are independently selected from the group consisting of C₁ - C₈ alkyl, C₄ - C₈ cycloalkyl, aryl, and C₇ - C₉ alkaryl, or R₁, R₂ and NH together form a C₃ - C₇ ring, n is 1, 2 or 3, and [X]ⁿ⁻ is selected from the group consisting of C₁-C₃ organic carboxyl, CO₃²⁻, HCO₃⁻, CT, NO₃⁻, SO₄²⁻, PO₄³⁻, HPO₄²⁻ and H₂PO₄⁻.

2. A method for preparing a tertiary amine with a secondary amine as raw material, **characterized in that,** the method comprises the step of reacting a secondary amine as raw material with an acid to generate a protic ionic liquid, and the step of reacting the protic ionic liquid with an aldehyde under the action of a reducing agent to generate a tertiary amine.

3. The method for preparing a tertiary amine with a secondary amine as raw material according to claim 2, **characterized in that,** the secondary amine has a carbon number below 30, preferable below 25, and further preferably below 20; and/or, the secondary amine is an aliphatic secondary amine and/or an aromatic secondary amine and/or a cyclic secondary amine; and/or, the acid is selected from the group consisting of organic carboxylic acids, inorganic acids, and combinations thereof; and/or, the aldehyde is a monoaldehyde, a dialdehyde, or an aldehyde polymer.

4. The method for preparing a tertiary amine with a secondary amine as raw material according to claim 2, **characterized in that,** the acid is selected from the group consisting of C₁ - C₃ organic carboxylic acids, hydrochloric acid, sulfuric acid, carbonic acid, phosphoric acid, nitric acid, and combinations thereof; preferably, the acid is selected from the group consisting of formic acid, acetic acid, propionic acid, oxalic acid, malonic acid, carbonic acid, hydrochloric acid, sulfuric acid, phosphoric acid, nitric acid, and combinations thereof.

5. The method for preparing a tertiary amine with a secondary amine as raw material according to claim 2, **characterized in that,** the reducing agent is selected from the group consisting of formic acid, sodium formate, oxalic acid, sodium oxalate, triphenyl silane, and combinations thereof; preferably, the reducing agent is selected from the group consisting of formic acid, sodium formate, oxalic acid, and sodium oxalate.

6. The method for preparing a tertiary amine with a secondary amine as raw material according to claim 2, **characterized in that,** the molar ratio of the secondary amine as raw material to the acid is 0.2 - 1.5, preferably 0.3 - 1.4, especially preferably 0.4 - 1.2; and/or, the molar ratio of the aldehyde to the protic ionic liquid is 0.2 - 5.0, preferably 0.3 - 3.0, especially preferably 0.4 - 2.0; and/or, the molar ratio of the reducing agent to the protic ionic liquid is 1.0 - 5.0, preferably 1.0 - 2.0.

7. The method for preparing a tertiary amine with a secondary amine as raw material according to claim 2, **characterized in that,** the step of reacting a secondary amine as raw material with an acid to generate a protic ionic liquid is carried out at room temperature and atmospheric pressure.

8. The method for preparing a tertiary amine with a secondary amine as raw material according to claim 2, **characterized in that,** the step of reacting the protic ionic liquid with an aldehyde under the action of a reducing agent to generate a tertiary amine is carried out at a pressure of 0 - 5.0 MPa, preferably 0 - 3.0 MPa, and particularly preferably 0 - 2.0 MPa; and/or, the step of reacting the protic ionic liquid with an aldehyde under the action of a reducing agent to generate a tertiary amine is carried out at a temperature of 60 - 200°C, preferably 120 - 160°C; and/or, the reaction time of the protic ionic liquid and the aldehyde under the action of a reducing agent is 5 - 300 min, preferably 30 - 120 min.

9. The method for preparing a tertiary amine with a secondary amine as raw material according to any one of claims 2 to 8, **characterized in that,** the method comprises the following steps:
1) dropwise adding the acid to the secondary amine or the secondary amine to the acid to generate the protic ionic liquid;
2) adding the protic ionic liquid and the reducing agent to a reactor, and adding the aldehyde to the reactor;
3) introducing nitrogen to the reaction system to a pressure of 0 - 5.0 MPa, heating to 60 - 200°C, and reacting for 5-300 min;
4) after the reaction is completed, purifying the reaction system to give the tertiary amine.

10. The method for preparing a tertiary amine with a secondary amine as raw material according to claim 9, **characterized in that,** in step 1), the acid is added dropwise to an aqueous solution of the secondary amine to generate the protic ionic liquid; and/or, in step 1), the acid is added dropwise to the secondary amine in the form of an aqueous solution of the acid; and/or, in step 2), the aldehyde is added at once, intermittently, or continuously into the reactor; and/or, in step 2), the reducing agent is added at once, intermittently, or continuously into the reactor; and/or, in step 4), the purification can be carried out through atmospheric distillation or reduced pressure distillation.

11. The method for preparing a tertiary amine with a secondary amine as raw material according to any one of claims 2 to 8, **characterized in that,** the structural formula of the secondary amine is 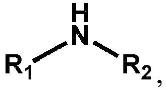 where R₁ and R₂ are independently selected from the group consisting of C₁ - C₈ alkyl, C₄ - C₈ cycloalkyl, aryl, and C₇ - C₉ alkaryl, or R₁, R₂ and NH together form a C₃ - C₇ ring; and/or the aldehyde is selected from the group consisting of monoaldehydes with a structural formula 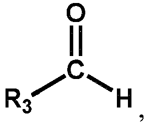 polymers of monoaldehydes with a structural formula 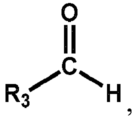 and dialdehydes with a structural formula 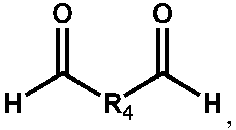 where R₃ is selected from the group consisting of H, C₁ - C₆ alkyl, and hydroxy substituted C₁ - C₆ alkyl, and R₄ is a single bond or C₁ - C₄ alkylidene.

12. The method for preparing a tertiary amine with a secondary amine as raw material according to any one of claims 2 to 8, **characterized in that,** the structural formula of the protic ionic liquid is 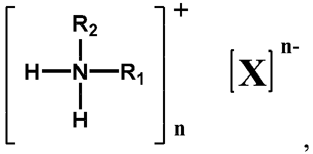 where R₁ and R₂ are independently selected from the group consisting of C₁ - C₈ alkyl, C₄ - C₈ cycloalkyl, aryl, and C₇ - C₉ alkaryl, or R₁, R₂ and N together form a C₃ - C₇ ring, n is 1, 2 or 3, and [X]ⁿ⁻ is selected from the group consisting of C₁-C₃ organic carboxyl, CO₃²⁻, HCO₃⁻, Cl, NO₃⁻, SO₄²⁻, PO₄³⁻, HPO₄²⁻ and H₂PO₄⁻.

13. The method for preparing a tertiary amine with a secondary amine as raw material according to claim 11, **characterized in that,** R₁ and R₂ are independently selected from the group consisting of methyl, ethyl, n-propyl, isopropyl, n-butyl, isobutyl, sec-butyl, tert-butyl, n-pentyl, isopentyl, sec-pentyl, neopentyl, 1,2-dimethylpropyl, n-hexyl, isohexyl, sec-hexyl, n-heptyl, isoheptyl, sec-heptyl, n-octyl, isooctyl, sec-octyl, 2-ethylhexyl, cyclopropyl, cyclobutyl, cyclopentyl, cyclohexyl, cycloheptyl, cyclooctyl, phenyl, 2-naphthyl, 2-methyl phenyl, 3-methyl phenyl, 4-methyl phenyl, 2,4-dimethyl phenyl, 2,5-dimethyl phenyl, 2,6-dimethyl phenyl, 3,4-dimethyl phenyl, 3,5-dimethyl phenyl, 2,3,4-trimethyl phenyl, 2,3,5-trimethyl phenyl, 2,3,6-trimethyl phenyl, 2,4,6-trimethyl phenyl, 2-ethyl phenyl, 3-ethyl phenyl, 4-ethyl phenyl, 2-n-propyl phenyl, 3-n-propyl phenyl, and 4-n-propyl phenyl, or R₁, R₂ and NH together form pyrrole, piperidine, morpholine, piperazine, N-methylpiperazine, hexamethyleneimine or azetidine; and/or, R₃ is selected from the group consisting of H, methyl, ethyl, n-propyl, isopropyl, n-butyl, isobutyl, n-pentyl, isopentyl, n-hexyl, isohexyl, hydroxymethyl, hydroxyethyl, and hydroxypropyl; and/or, R₄ is selected from the group consisting of a single bond, methylene, ethylidene, propylidene, and butylidene; and/or, the aldehyde is selected from the group consisting of acetaldehyde, paraldehyde, metaldehyde, propionaldehyde, glycolaldehyde, glyoxal, malondialdehyde, succinaldehyde, glutaraldehyde and adipaldehyde.
